# EUROPÄISCHE PATENTSCHRIFT

(11) **EP 0 814 906 B1**
(45) Veröffentlichungstag und Bekanntmachung des Hinweises auf die Patenterteilung: **24.05.2000**
(21) Anmeldenummer: 96907340.2
(22) Anmeldetag: 04.03.1996
(51) Int. Cl.: B01J 27/18, B01J 23/96, B01J 37/34, C07C 45/38

(54) **VERFAHREN ZUR HERSTELLUNG VON PHOSPHORDOTIERTEN SILBERKATALYSATOREN**
PROCESS FOR PREPARING PHOSPHORUS-DOPED SILVER CATALYSTS
PROCEDE DE PREPARATION DE CATALYSEURS EN ARGENT DOPES AU PHOSPHORE

(30) Priorität: 15.03.1995 DE 19509359
(43) Veröffentlichungstag der Anmeldung: 07.01.1998
(73) Patentinhaber: BASF AKTIENGESELLSCHAFT, 67056 Ludwigshafen (DE)
(72) Erfinder: DIERCKS, Rainer, D-67141 Neuhofen (DE); SCHÖNAICH, Günther, D-67141 Neuhofen (DE)
(86) Internationale Anmeldenummer: EP9600901
(87) Internationale Veröffentlichungsnummer: WO9628249

(56) Entgegenhaltungen:
- EP-A- 0 091 060
- EP-A- 0 104 666
- EP-A- 0 447 267
- EP-A- 0 467 169
- LU-A- 65 795

## Beschreibung

Die vorliegende Erfindung betrifft ein Verfahren zur Herstellung von phosphordotierten Silberkatalysatoren aus mit Phosphorverbindungen verunreinigtem Silber.

Verfahren zur Herstellung von Silberkatalysatoren, die für die Oxidation von Methanol zu Formaldehyd geeignet sind, sind allgemein bekannt (vgl. Ullmann's Enzyklopädie der technischen Chemie, 3. Auflage, Urban und Schwarzenberg, München-Berlin, 1956, 7. Band, S. 660 bis 663). Nach diesem Verfahren wird Silber in einer elektrolytischen Zelle anodisch zu Silberionen oxidiert und kathodisch wieder zu Silber reduziert. Als Elektrolyt wird eine wäßrige Lösung aus Silbernitrat und Salpetersäure empfohlen. Das an der Kathode gebildete grobkristalline Silber eignet sich als Katalysator zur Formaldehydsynthese aus Methanol.

Nach der Lehre von DE-A-1166171 lassen sich bei der Oxidation von Methanol zu Formaldehyd besonders hohe Ausbeuten erzielen, wenn man einen Silberkatalysator einsetzt, der nach dem folgenden Verfahren hergestellt wird: Silber, das gegebenenfalls zunächst auf 500 bis 600°C in einem Strom sauerstoffhaltiger Gase erhitzt wurde, wird mindestens einer zweimaligen Elektrolyse unterworfen, wobei die Stromdichte bei der ersten Elektrolyse bzw. den ersten Elektrolysen oberhalb von 250, insbesondere zwischen 300 und 500 A/m² Kathodenfläche und bei der letzten Elektrolyse unterhalb von 250, insbesondere zwischen 100 und 200 A/m² Kathodenfläche liegt.

Aus der EP-A-0104666 ist bekannt, daß die Selektivität bei der Herstellung von Formaldehyd aus Methanol mit Hilfe eines Silberkatalysators gesteigert werden kann, wenn neben dem Silberkatalysator kleine Mengen Phosphorverbindungen als Promotoren verwendet werden.

Vorteilhafte Effekte, die bei der Verwendung von Phosphorverbindungen als Promotoren zur Oxidation von Methanol zu Formaldehyd in Gegenwart eines Silberkatalysators auftreten, sind weiterhin aus der CN-A-85100530, EP-A-0467169 und der JP-A-38227/83 bekannt.

Die EP-A-0467169 beschreibt die Herstellung eines Katalysator Festbetts, das aus Schichten von Silberkristallen aufgebaut ist, die ein pulverförmiges phosphorhaltiges Salz als Promotor enthalten. Mit Hilfe dieses phosphordotierten Silberkatalysator-Festbett lassen sich bei der Herstellung von Formaldehyd aus Methanol besonders hohe Ausbeuten und Umsätze erzielen.

Dem kommerziellen Einsatz der Verfahren zur Herstellung von Formaldehyd unter Verwendung von Phosphorverbindungen als Promotoren steht bisher entgegen, daß keine wirtschaftlichen Verfahren zur Regenerierung der bei diesen Verfahren anfallenden verbrauchten Silberkatalysatoren, die mit Phosphorverbindungen verunreinigt sind, bekannt sind. Ein Verfahren zur Regenerierung dieser Katalysatoren ist für die Wirtschaftlichkeit der Formaldehydherstellung wichtig, da die Katalysatoren nach ca. 8 Betriebswochen ihre Aktivität so weit verloren haben, daß eine unbefriedigende Raum-Zeit-Ausbeute die Folge ist.

Werden die mit Phosphorverbindungen verunreinigten Silberkatalysatoren nach einem der vorbeschriebenen Verfahren zur Herstellung von Silberkatalysatoren regeneriert, so erhält man Silberkatalysatoren, die für eine wirtschaftliche Herstellung von Formaldehyd im großtechnischen Maßstab aus den folgenden Gründen wenig geeignet sind:

Wenn ein Silberkatalysator, der in einer üblichen kontinuierlich betriebenen Anlage zur industriellen Produktion von Formaldehyd eingesetzt wird, seine Aktivität verliert, ist es erforderlich, den Produktionsprozeß zu unterbrechen, um den Katalysator der Anlage entnehmen und gegen regenerierten Katalysator mit höherer Aktivität austauschen zu können. Beim erneuten Starten des Syntheseprozesses muß der Gasstrom der Ausgangsprodukte, der über den Katalysator geleitet wird, vorgewärmt werden. Im Fall von regenerierten Katalysatoren, die nicht mit Phosphorverbindungen als Promotoren eingesetzt worden waren, sind hierzu Temperaturen von ca. 300 bis 350°C erforderlich. Ist die Reaktion erst einmal gestartet, erübrigt sich ein weiteres Vorwärmen des Gasstromes, weil sich bei der Formaldehydsynthese die Reaktionszone (Katalysator) aufgrund der freiwerdenden Reaktionswärme auf Temperaturen von ca. 700°C erhitzt.

Beim Einsatz von regenerierten Silberkatalysatoren, die zusammen mit Phosphorverbindungen als Promotoren eingesetzt worden waren, muß der Gasstrom der Ausgangsprodukte, damit die Reaktion in Gang kommt, auf Temperaturen von mindestens 700°C vorgewärmt werden.

Das Vorwärmen des Gasstroms auf eine derartig hohe Temperatur erfordert einen hohen technischen Aufwand. Die hierfür erforderlichen Vorrichtungen sind in den üblicherweise für die industrielle Produktion von Formaldehyd verwendeten Anlagen nicht vorhanden.

Aus Gmelins Handbuch der anorganischen Chemie, Verlag Chemie GmbH 1970, 8. Auflage, Teil A2, System-Nummer 61, S. 34 bis 35, ist bekannt, reines Silber herzustellen, indem man Rohsilber durch Schmelzverfahren (Treibprozeß) möglichst weitgehend anreichert und das Silber anschließend weiter elektrolytisch reinigt. Dabei wird das Silber in einer salpetersauren Silbernitratlösung anodisch aufgelöst und kathodisch abgeschieden.

Der Treibprozeß, bei dem Rohsilber in Gegenwart von Luft auf Temperaturen von 900 bis 1100°C erhitzt wird (vgl. loc. cit. S. 7 bis 9) wird jedoch nur für Rohsilber empfohlen, das mit Pb, Cu, As, Sb, Zn, Se und Te verunreinigt ist.

Der vorliegenden Erfindung lag deshalb die Aufgabe zugrunde, ein Verfahren zur Herstellung von phosphordotierten Silberkatalysatoren bereitzustellen, welches die Nachteile des Standes der Technik nicht aufweist.

Diese Aufgabe wurde erfindungsgemäß durch ein Verfahren gelöst, welches dadurch gekennzeichnet ist, daß man
a) das mit Phosphorverbindungen verunreinigte Silber auf Temperaturen, bei denen das Silber flüssig ist, erhitzt und anschließend auf Temperaturen unterhalb des Schmelzpunktes abkühlt,
b) aus dem nach Stufe a) erhaltenen Silber Silberkristalle herstellt, indem man es in einer Elektrolysezelle mit einem wäßrigen Elektrolyten anodisch zu Silberionen oxidiert und die Silberionen kathodisch wieder zu elementarem Silber reduziert und
c) die Silberkristalle mit einer in feinverteilter Form vorliegenden Phosphorverbindung mit einer Schmelz- bzw. Zersetzungstemperatur von mehr als 500°C (Phosphorverbindung P) in Kontakt bringt.

Als Ausgangsmaterial für das erfindungsgemäße Verfahren kommt insbesondere Silber in Betracht, das ca. 0,01 bis 1000 ppm, bevorzugt 1 bis 100 ppm Phosphor oder Phosphorverbindungen enthält, berechnet als elementarer Phosphor, jedoch praktisch frei von Verunreinigungen wie Pb, Cu, As, Sb, Zn, Se, Te und Edelmetallen ist. Derartig verunreinigtes Silber fällt beispielsweise an, wenn Silber als Katalysator zusammen mit phosphorhaltigen Promotoren zur Oxidation von Methanol zu Formaldehyd verwendet wird. Verfahren, bei denen derartig verunreinigte Katalysatoren, die regeneriert werden müssen, anfallen, sind beispielsweise aus der EP-A-0104666, CN-A-85100530 und der JP-A-38227/83 bekannt.

Die Katalysatoren, die bei diesen Verfahren eingesetzt werden, erhält man beispielsweise, indem man Silber mit Lösungen von Phosphor oder organischen Phosphorverbindungen, z.B. Triphenylphosphin oder anorganische Phosphorverbindungen, z.B. Orthophosphorsäure imprägniert oder mit Phosphor bedampft.

Besonders bevorzugt setzt man als mit Phosphor verunreinigtes Silber einen verbrauchten phosphordotierten Silberkatalysator ein, wie er bei der Herstellung von Formaldehyd durch oxidative Dehydrierung von Methanol nach dem in der EP-A-0467169 beschriebenen Verfahren anfällt.

Bei diesem Verfahren setzt man einen phophordotierten Ausgangs-Silberkatalysator, der erhältlich ist, indem man Silberkristalle, die praktisch frei von Phosphorverbindungen und sonstigen Verunreinigungen sind, mit einem phosphorhaltigen Salz, z.B. Phosphaten oder Polyphosphaten von Alkali- oder Erdalkalimetallen in Kontakt bringt, nach einem allgemein bekannten Verfahren (vgl. Ullmann's Enzyklopädie der technischen Chemie, 3. Auflage, Urban und Schwarzenberg, München-Berlin, 1956, 7. Band, S. 660 bis 661) für die oxidative Dehydrierung von Methanol zu Formaldehyd ein.

In Schritt (a) des erfindungsgemäßen Verfahrens erfolgt das Aufschmelzen des Silbers bevorzugt in Anwesenheit von 1 bis 100 Gew.-%, bezogen auf das Silber, einer Verbindung oder einer Mischung von Verbindungen, ausgewählt aus der Gruppe der Erdalkalimetalloxide, bevorzugt Calciumoxid, Siliziumdioxid oder Aluminiumoxid.

Besonders geeignet sind hierzu ternäre und binare Gemische aus Siliziumdioxid, Aluminiumoxid und Calciumoxid, die besonders bevorzugt als eutektische Gemische eingesetzt werden, um den Schmelzpunkt möglichst niedrig zu erhalten.

Das Silber wird im allgemeinen in Gegenwart von Sauerstoff für 0,5 bis 10 min auf Temperaturen, bei denen das Silber unter Normalbedingungen flüssig ist, bevorzugt auf Temperaturen von 960,8 bis 1500°C erhitzt. Falls das Aufschmelzen des Silbers in Gegenwart von Zuschlägen erfolgt, liegt die Temperatur günstigerweise oberhalb der Schmelztemperatur dieser Zuschläge.

Anschließend wird das Silber abgekühlt. Es ist von Vorteil, das geschmolzene Silber hierzu in Wasser zu gießen, um etwaige wasserlösliche Verunreinigungen abzutrennen.

In dem folgenden Schritt (b) wird das Silber einer Elektrolyse unterworfen. Dabei wird das Silber in einer Elektrolysezelle mit einem wäßrigen Elektrolyten anodisch zu Silberionen oxidiert und kathodisch wieder zu elementarem Silber reduziert. Hierfür eignen sich Verfahren, wie sie für die elektrolytische Reinigung von Silber im allgemeinen bekannt sind (vgl. Hollemann-Wiberg, Lehrbuch der anorganischen Chemie, 91.-100. Auflage, Verlag Walter de Gruyter 1985, S. 1012-1013).

Elementares Silber in Form von Silberkristallen, die besonders als Katalysatoren bei der Formaldehydsynthese geeignet sind, erhält man insbesondere dann, wenn man die Elektrolyse nach dem in der deutschen Patentschrift 1166171 beschriebenen Verfahren durchführt.

Bevorzugt wird als Elektrolyt eine wäßrige Silbernitratlösung eingesetzt. Diese Silbernitratlösung hat im allgemeinen einen pH-Wert von 1 bis 4 und enthält 1 bis 5 Gew.-% Silber. Der pH-Wert wird günstigerweise mit Salpetersäure eingestellt.

Als Elektroden dienen die üblicherweise bei der Elektrolyse von Silber verwendeten Elektroden. Geeignete Anoden sind Säcke, in die das zu oxidierende Silber im allgemeinen als Granulat oder als Pulver eingefüllt worden ist. Als Kathoden kommen insbesondere Silberbleche in Betracht.

Die Elektrolyse wird günstigerweise bei Stromdichten von 80 bis 500 A/m² Kathodenfläche und Elektrolyttemperaturen von 10 bis 30°C durchgeführt.

Um diese Stromdichten zu erreichen, sind bei den meisten Elektrolysezellen Spannungen von 1 bis 15 Volt erforderlich.

Es empfiehlt sich, die an der Kathode gebildeten Silberkristalle fortwährend von der Kathode zu entfernen. Es werden im allgemeinen Silberkristalle mit einer Korngröße von 0,2 bis 5 mm erhalten.

Meistens reicht eine einmalige Elektrolyse aus, um brauchbare Silberkristalle zu erhalten.

Die Silberkristalle werden in Schritt (c) mit einer in feinverteilter Form vorliegenden Phosphorverbindung mit einer Schmelz- bzw. Zersetzungstemperatur von mehr als 500°C (Phosphorverbindung P) in Kontakt gebracht. Bevorzugt sind pulverförmige phosphorhaltige Salze, welche eine Schmelz- bzw. Zersetzungstemperatur oberhalb von 800°C aufweisen.

Als Salze kommen z.B. anorganische Phosphate von Alkali- und Erdalkalimetallen, Schwermetallen, z.B. Ag, Zn und Fe oder von Bor und Ammonium in Betracht.

Bevorzugt sind Phosphate oder Pyrophosphate von Alkali oder Erdalkalimetallen, z.B. von Na₄P₂O₇, Li₃PO₄, Mg₃(PO₄)₂, Ca₃(PO₄)₂.

Die Korngröße der eingesetzten Verbindungen (P) ist nicht kritisch, im allgemeinen beträgt sie ca. 1 mm bis 1 µm.

Bevorzugt enthält der phosphordotierte Silberkatalysator pro cm³ Silberkristalle 0,1 bis 0,0001 g, besonders bevorzugt 0,01 bis 0,001 g der Verbindung (P), berechnet als elementarer Phosphor.

Im allgemeinen geht man bei der Herstellung der phosphordotierten Silberkatalysatoren, aus den Silberkristallen so vor, wie es in EP-A-0467169 beschrieben ist. Nach diesem Verfahren ordnet man die Silberkristalle zu einem aus mehreren Schichten der Silberkristalle bestehenden Katalysator-Festbett an, wie es beispielsweise aus der DE-A-2322757 bekannt ist und bringt auf eine oder mehrere dieser Schichten die Verbindung (P) in Form eines Pulvers auf, wobei man ein phosphordotiertes Silberkatalysator-Festbett erhält.

Im allgemeinen besteht ein phosphordotiertes Silberkatalysatoren-Festbett aus 4 bis 9 Schichten von Silberkristallen und weist eine Gesamtschichtdicke von 5 bis 50 mm auf.

Die Menge an Phosphorverbindung (P), berechnet als elementarer Phosphor, beträgt im allgemeinen 0,1 bis 100 mg, vorzugsweise 0,5 bis 50 mg pro cm² Querschnittsfläche des phosphordotierten Silberkatalysatoren-Festbetts.

Besonders einfach kann man ein phosphordotiertes Silberkatalysatoren-Festbett herstellen, indem man ein aus den Silberkristallen bestehendes Katalysator-Festbett so in einen Rohrreaktor einbaut, daß die Schichten senkrecht zur Strömungsrichtung eines Gases angeordnet sind, das man durch den Rohrreaktor leitet und anschließend die Verbindung (P) auf die oberste Schicht der Silberkristalle streut. Zweckmäßigerweise ist die Querschnittsfläche des Rohrreaktors und des phosphordotierten Silberkatalysatoren-Festbetts in etwa gleich groß.

Diese mit einem phosphordotierten Silberkatalysatoren-Festbett ausgestatteten Rohrreaktoren eignen sich für die Herstellung von Formaldehyd durch oxidative Dehydrierung von Methanol, indem man Methanol und Sauerstoff bei einer Temperatur von üblicherweise 500 bis 750°C durch den Rohrreaktor leitet. Dieses Verfahren ist beispielsweise aus Ullmann's Enzyklopädie der technischen Chemie, 3. Auflage, Urban und Schwarzenberg, München-Berlin, 1956, 7. Band, S. 660 bis 661 oder aus der EP-A-0467169 bekannt.

Die auf diese Weise hergestellten phosphordotierten Silberkatalysatoren zeichnen sich insbesondere dadurch aus, daß in Gegenwart der Katalysatoren ein Vorwärmen der Reaktanden auf relativ niedrige Temperaturen ausreicht, um den Formaldehyd-Herstellungsprozess, mit dem Formaldehyd mit einem hohen Umsatz und hoher Selektivität hergestellt werden kann, zu starten.

### Beispiele

### A. Herstellung der phosphordotierten Silberkatalysatoren

### Beispiel 1

Ein Silberkatalysator, der gemäß dem Beispiel mit der Versuchs-Nr. 3 aus EP-A-0467169 hergestellt worden war (mit Na₄P₂O₇ beaufschlagt) wurde 2000 Betriebsstunden zur Formaldehydsynthese eingesetzt.

Zur Aufarbeitung wurden 50 g des Katalysators zunächst mit destilliertem Wasser lh gewaschen. Der Gehalt an Phosphorverbindungen betrug, gemessen als elementarer Phosphor, 27 Gew.-ppm.

Der Katalysator wurde 15min auf 1200°C erhitzt und anschließend in destilliertes Wasser gegossen, wonach der Gehalt an Phosphorverbindungen weniger als 1 ppm (Nachweisgrenze) betrug.

Aus dem Silber wurde danach nach dem Möbius-Verfahren elektrolytisch ein neuer Silberkatalysator hergestellt (vgl. Ullmann's Enzyklopädie der technischen Chemie, 3. Auflage, Urban und Schwarzenberg, München-Berlin, 1956, 7. Band, S. 660 bis 661). Elektrolysebedingungen: Stromdichte 120 A/m²; Elektrolyttemperatur 27°C; wäßrige Silbernitratlösung mit einem pH-Wert von 2,5 und einer Silberkonzentration von 3 g/l.

### Beispiel 2

Die Herstellung des Silberkatalysators erfolgte wie in Beispiel 1 beschrieben, jedoch wurde der verbrauchte Katalysator in Gegenwart von 25 Gew.-%, bezogen auf den Katalysator, einer Mischung aus Aluminiumoxid und Calciumoxid im Gewichtsverhältnis 1:1 aufgeschmolzen.

### Vergleichsbeispiel

Die Herstellung des Silberkatalysators erfolgte wie im Beispiel der deutschen Patentschrift 1166171.

### B. Eigenschaften der Silberkatalysatoren

Die Silberkatalysatoren, die gemäß Beispiel 1 und 2 sowie dem Vergleichsbeispiel hergestellt worden waren, wurden, wie im Beispiel der EP-A-0467169 beschrieben, zusammen mit Na₄P₂O₇ als Promotor in dem dort beschriebenen Versuchsreaktor eingesetzt. Die Versuchsbedingungen wurden ansonsten wie bei Versuch Nr. 3 gewählt. Mit den Katalysatoren gemäß Beispiel 1 und 2 konnte das Ergebnis des Versuchs Nr. 3 reproduziert werden. Die Reaktion sprang bei einer Temperatur der Ausgangsprodukte von 330°C an.

Bei Verwendung des Katalysators des Vergleichsbeispiels sprang die Reaktion trotz Erwärmung der Ausgangsprodukte auf 550°C nicht an.

## Patentansprüche

1. Verfahren zur Herstellung von phosphordotierten Silberkatalysatoren aus mit Phosphorverbindungen verunreinigtem Silber, dadurch gekennzeichnet, daß man
a) das mit Phosphorverbindungen verunreinigte Silber auf Temperaturen, bei denen das Silber flüssig ist, erhitzt und anschließend auf Temperaturen unterhalb des Schmelzpunktes abkühlt,
b) aus dem nach Stufe (a) erhaltenen Silber Silberkristalle herstellt, indem man es in einer Elektrolysezelle mit einem wäßrigen Elektrolyten anodisch zu Silberionen oxidiert und die Silberionen kathodisch wieder zu elementarem Silber reduziert, und
c) die Silberkristalle mit einer in feinverteilter Form vorliegenden Phosphorverbindung mit einer Schmelz- bzw. Zersetzungstemperatur von mehr als 500°C (Phosphorverbindung P), in Kontakt bringt.

2. Verfahren nach Anspruch 1, dadurch gekennzeichnet, daß man das mit Phosphorverbindungen verunreinigte Silber in Gegenwart von 1 bis 100 Gew.-%, bezogen auf das Silber, einer Verbindung oder einer Mischung von Verbindungen, ausgewählt aus der Gruppe der Erdalkalimetalloxide, Siliziumdioxid, Aluminiumoxid, auf Temperaturen oberhalb des Schmelzpunktes dieser Verbindungen oder der entsprechenden Mischungen erhitzt.

3. Verfahren nach Anspruch 1 oder 2, dadurch gekennzeichnet, daß man das flüssige Silber zum Abkühlen in deionisiertes Wasser gießt.

4. Verfahren nach den Ansprüchen 1 bis 3, dadurch gekennzeichnet, daß man als Elektrolyt eine wäßrige Silbernitratlösung einsetzt.

5. Verfahren nach den Ansprüchen 1 bis 4, dadurch gekennzeichnet, daß die Stromdichte bei der Elektrolyse 80 bis 500 A/m² Elektrodenfläche beträgt.

6. Verfahren nach den Ansprüchen 1 bis 5, dadurch gekennzeichnet, daß man als Verbindung (P) ein Alkali- oder Erdalkaliphosphat oder ein Alkali- oder Erdalkalipyrophosphat einsetzt.

7. Verfahren nach den Ansprüchen 1 bis 6, dadurch gekennzeichnet, daß man als mit Phosphor verunreinigtes Silber einen verbrauchten phosphordotierten Silberkatalysator einsetzt, der erhältlich ist, indem man einen phophordotierten Ausgangs-Silberkatalysator für die oxidative Dehydrierung von Methanol zu Formaldehyd verwendet.

8. Verfahren nach den Ansprüchen 1 bis 7, dadurch gekennzeichnet, daß man ein phosphordotiertes Silberkatalysator-Festbett herstellt, indem man die Silberkristalle zu einem aus einer oder mehreren Schichten Silberkristalle bestehenden Silberkatalysator-Festbett anordnet und auf eine oder mehrere dieser Schichten die Phosphorverbindung (P) streut.

9. Verfahren nach Anspruch 8, dadurch gekennzeichnet, daß man die Silberkristalle zu einem aus 4 bis 9 Schichten bestehenden Silberkatalysator-Festbett anordnet.

10. Verfahren nach Anspruch 8 oder 9, dadurch gekennzeichnet, daß man die Silberkristalle zu einem Silberkatalysator-Festbett mit einer Gesamtschichtdicke von 5 bis 50 mm anordnet.

## Claims

1. A process for preparing phosphorus-doped silver catalysts from silver contaminated with phosphorus compounds, which comprises
a) heating the silver which is contaminated with phosphorus compounds to temperatures at which the silver is liquid and subsequently cooling to temperatures below the melting point,
b) preparing silver crystals from the silver obtained in stage a) by anodically oxidizing it to silver ions in an electrolysis cell with an aqueous electrolyte, and cathodically reducing the silver ions again to elemental silver, and
c) contacting the silver crystals with a finely divided phosphorus compound with a melting point or decomposition temperature above 500°C (phosphorus compound P).

2. A process as claimed in claim 1, wherein the silver contaminated with phosphorus compounds is heated in the presence of from 1 to 100 % by weight, based on the silver, of a compound or of a mixture of compounds selected from the group of alkaline earth metal oxides, silicon dioxide, aluminum oxide, to temperatures above the melting point of these compounds or of the corresponding mixtures.

3. A process as claimed in claim 1 or 2, wherein the liquid silver is cooled by pouring into deionized water.

4. A process as claimed in any of claims 1 to 3, wherein an aqueous silver nitrate solution is used as electrolyte.

5. A process as claimed in any of claims 1 to 4, wherein the current density in the electrolysis is from 80 to 500 A/m² of electrode area.

6. A process as claimed in any of claims 1 to 5, wherein an alkali metal or alkaline earth metal phosphate or an alkali metal or alkaline earth metal pyrophosphate is used as compound (P).

7. A process as claimed in any of claims 1 to 6, wherein a used phosphorus-doped silver catalyst which is obtainable by using an initial phosphorus-doped silver catalyst for the oxidative dehydrogenation of methanol to formaldehyde is employed as phosphorus-contaminated silver.

8. A process as claimed in any of claims 1 to 7, wherein a phosphorus-doped fixed bed silver catalyst is prepared by arranging the silver crystals to give a fixed bed silver catalyst which consists of one or more layers of silver crystals, and distributing the phosphorus compound (P) on one or more of these layers.

9. A process as claimed in claim 8, wherein the silver crystals are arranged to give a fixed bed silver catalyst consisting of from 4 to 9 layers.

10. A process as claimed in claim 8 or 9, wherein the silver crystals are arranged to give a fixed bed silver catalyst with a total layer thickness of from 5 to 50 mm.

## Revendications

1. Procédé de préparation de catalyseurs à l'argent dopés au phosphore à partir d'argent contaminé par des composés du phosphore, caractérisé en ce que
a) l'argent contaminé par des composés du phosphore est chauffé à des températures auxquelles l'argent est liquide, puis refroidit à des températures inférieures au point de fusion,
b) on prépare des cristaux d'argent à partir de l'argent obtenu à l'issue de l'étape a), en l'oxydant en ions argent à l'anode dans une cellule d'électrolyse ayant un électrolyte aqueux, et en réduisant les ions argent en l'élément argent à la cathode, et
c) on met les cristaux d'argent en contact avec un composé du phosphore se trouvant sous forme finement divisée et ayant une température de fusion ou de décomposition supérieure à 500°C (composé du phosphore P).

2. Procédé selon la revendication 1, caractérisé en ce que l'on chauffe l'argent contaminé par des composés du phosphore en présence de 1-100% en poids, par rapport à l'argent, d'un composé ou d'un mélange de composés, choisis dans le groupe formé par les oxydes de métaux alcalino-terreux, le dioxyde de silicium et l'oxyde d'aluminium, à des températures supérieures au point de fusion de ces composés ou des mélanges correspondants.

3. Procédé selon la revendication 1 ou 2, caractérisé en ce que l'on verse l'argent liquide dans de l'eau désionisée pour le refroidir.

4. Procédé selon l'une quelconque des revendications 1 à 3, caractérisé en ce que l'on utilise une solution aqueuse de nitrate d'argent en tant qu'électrolyte.

5. Procédé selon l'une quelconque des revendications 1 à 4, caractérisé en ce que la densité de courant lors de l'électrolyse s'élève à 80-500 A/m² de surface d'électrode.

6. Procédé selon l'une quelconque des revendications 1 à 5, caractérisé en ce que l'on utilise, en tant que composé P, un phosphate de métal alcalin ou alcalino-terreux ou pyrophosphate de métal alcalin ou alcalino-terreux.

7. Procédé selon l'une quelconque des revendications 1 à 6, caractérisé en ce que l'on utilise, en tant qu'argent contaminé par des composés de phosphore, un catalyseur usagé à l'argent dopé au phosphore, pouvant être obtenu par l'utilisation d'un catalyseur de départ à l'argent dopé au phosphore pour la déshydrogénation oxydante du méthanol en formaldéhyde.

8. Procédé selon l'une quelconque des revendications 1 à 7, caractérisé en ce que l'on prépare un lit fixe de catalyseur à l'argent dopé au phosphore, en disposant les cristaux d'argent en un lit fixe de catalyseur à l'argent constitué d'une ou plusieurs couches de cristaux d'argent et on disperse le composé du phosphore (P) sur une ou plusieurs de ces couches.

9. Procédé selon la revendication 8, caractérisé en ce que l'on dispose les cristaux d'argent en un lit fixe de catalyseur à l'argent constitué de 4 à 9 couches.

10. Procédé selon la revendication 8 ou 9, caractérisé en ce que l'on dispose les cristaux d'argent en un lit fixe de catalyseur à l'argent ayant une épaisseur totale de 5-50 mm.
